# EUROPEAN PATENT APPLICATION

(11) **EP 4 393 490 A1**
(43) Date of publication of application: **03.07.2024**
(21) Application number: 22863434.1
(22) Date of filing: 30.08.2022
(51) Int. Cl.: A61K 31/4545, A61P 35/00

(54) **USE OF EZH2 INHIBITOR IN PREPARATION OF DRUG FOR TREATING T-CELL LYMPHOMA**

(30) Priority: 30.08.2021 CN 202111001663
(71) Applicant: Jiangsu Hengrui Pharmaceuticals Co., Ltd., Lianyungang, Jiangsu 222047 (CN)
(72) Inventor: ZHANG, Xiaojing, Lianyungang, Jiangsu 222047 (CN); WANG, Weiwei, Lianyungang, Jiangsu 222047 (CN)
(74) Representative: D Young & Co LLP
(86) International application number: PCT/CN2022/115763
(87) International publication number: WO 2023/030299

(57) **Abstract**

The present disclosure relates to a use of an EZH2 inhibitor in the preparation of drugs for the treatment of T-cell lymphoma. Specifically, it relates to the use of a compound shown in Formula (I) or a pharmaceutically acceptable salt thereof in the preparation of drugs for the treatment of T-cell lymphoma.

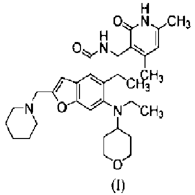

## Description

This application claims priority rights for Chinese patent application 2021110016631 with an application date of August 30, 2021. This application cites the full text of the aforementioned Chinese patent application.

### Technical Field

The present disclosure relates to a use of an EZH2 inhibitor for the preparation of drugs for the treatment of T-cell lymphoma, which belongs to the field of pharmaceutics.

### Background Art

Enhancer of zeste homolog 2 (EZH2) is the core component of histone methyltransferase PRC2 (polycomb repressive complex 2). It catalyzes the trimethylation of lysine 27 at the N-terminal of histone H3 (H3K27Me3), which triggers and maintains the transcriptional repression of chromatin, thus repressing target gene expression. Most of these target genes inhibit cell proliferation and promote cell differentiation, thus playing an important role in maintaining embryonic development and resisting cellular senescence.

Peripheral T-cell lymphoma (PTCL), also known as mature T-cell lymphoma, is a group of malignant proliferative diseases with obvious heterogeneity originating from mature T lymphocytes. Because the immune manifestations and functions of NK cells are similar to those of T cells, NK cell lymphoma and mature T-cell lymphoma are often classified in the same category.

At present, there is no standard treatment regimen for PTCL. The usual first-line treatment is CHOP or CHOP-like regimen, and the 5-year survival rate is only about 30%. For relapsed or refractory PTCL (rrPTCL), conducting clinical trials first is recommended in China and abroad. Other Class I experts recommend treatments including new drugs alone and in combination with chemotherapy, but the efficacy of traditional chemotherapy is not ideal. Currently, chidamide and pralatrexate are approved for the treatment of rrPTCL in China, with an ORR of 27-52% and a PFS of 2.1-4.8 months. However, there have been no available treatments for patients who relapse or are refractory after undergoing treatment with new drugs. In conclusion, the efficacy of traditional chemotherapy in relapsed or refractory PTCL is not ideal. Histone deacetylase (HDAC) inhibitors and folic acid inhibitors are approved for use in such patients, but the improvement of efficacy is limited. At present, more new and effective drugs need to be developed and marketed to improve the prognosis of patients with relapsed or refractory PTCL.

### Summary of the Invention

The present disclosure provides the use of a compound shown in Formula (I) or a pharmaceutically acceptable salt thereof in the preparation of drugs for the treatment of T-cell lymphoma:

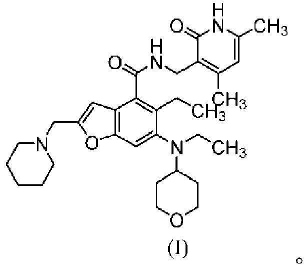

In some embodiments, the T-cell lymphoma of the present disclosure is peripheral T-cell lymphoma (mature T- and NK-cell lymphoma).

In some embodiments, the T-cell lymphoma of the present disclosure is angioimmunoblastic T-cell lymphoma (AITL), anaplastic large cell lymphoma (ALCL), peripheral T lymphoma-non-specific (PTCL-NOS), or NK/T-cell lymphoma (NKTCL).

In some embodiments, the T-cell lymphoma of the present disclosure is an angioimmunoblastic T-cell lymphoma or a peripheral T-lymphoma, not otherwise specified.

In some embodiments, the T-cell lymphoma of the present disclosure is angioimmunoblastic T-cell lymphoma.

In some embodiments, the T-cell lymphoma of the present disclosure is a peripheral T lymphoma, not otherwise specified.

In some embodiments, the T-cell lymphoma of the present disclosure is relapsed/refractory peripheral T-cell lymphoma.

In some embodiments, the T-cell lymphoma of the present disclosure is peripheral T-cell lymphoma which has undergone first-line chemotherapy.

In some embodiments, the T-cell lymphoma of the present disclosure is a peripheral T-cell lymphoma which has undergone at least one treatment selected from histone deacetylase inhibitors, folic acid metabolism inhibitors, or anti-CD30 monoclonal antibodies.

In some embodiments, the T-cell lymphoma of the present disclosure is a peripheral T-cell lymphoma which has undergone first-line chemotherapy and at least one treatment selected from histone deacetylase inhibitors, folic acid metabolism inhibitors, or anti-CD30 monoclonal antibodies.

In some embodiments, the administered dose of the compound shown in Formula (I) or a pharmaceutically acceptable salt thereof is selected from 1-800mg, administered once or twice a day.

In some embodiments, the administered dose of the compound shown in Formula (I) or a pharmaceutically acceptable salt thereof is selected from 2.5mg, 5.0mg, 7.5mg, 10.0mg, 12.5mg, 15.0mg, 17.5mg, 20.0mg, 22.5mg, 25.0mg, 27.5mg, 30.0mg, 32.5mg, 35.0mg, 37.5mg, 40.0mg, 42.5mg, 45.0mg, 47.5mg, 50.0mg, 52.5mg, 55.0mg, 57.5mg, 60.0mg, 62.5mg, 65.0mg, 67.5mg, 70.0mg, 72.5mg, 75.0mg, 77.5mg, 80.0mg, 82.5mg, 85.0mg, 87.5mg, 90.0mg, 92.5mg, 95.0mg, 97.5mg, 100.0mg, 102.5mg, 105.0mg, 107.5mg, 110.0mg, 112.5mg, 115.0mg, 117.5mg, 120.0mg, 122.5mg, 125.0mg, 127.5mg, 130.0mg, 132.5mg, 135.0mg, 137.5mg, 140.0mg, 142.5mg, 145.0mg, 147.5mg, 150.0mg, 152.5mg, 155.0mg, 157.5mg, 160.0mg, 162.5mg, 165.0mg, 167.5mg, 170.0mg, 172.5mg, 175.0mg, 177.5mg, 180.0mg, 182.5mg, 185.0mg, 187.5mg, 190.0mg, 192.5mg, 195.0mg, 197.5mg, 200.0mg, 202.5mg, 205.0mg, 207.5mg, 210.0mg, 212.5mg, 215.0mg, 217.5mg, 220.0mg, 222.5mg, 225.0mg, 227.5mg, 230.0mg, 232.5mg, 235.0mg, 237.5mg, 240.0mg, 242.5mg, 245.0mg, 247.5mg, 250.0mg, 252.5mg, 255.0mg, 257.5mg, 260.0mg, 262.5mg, 265.0mg, 267.5mg, 270.0mg,272.5mg,275.0mg,277.5mg,280.0mg,282.5mg,285.0mg,287.5mg,290.0mg, 292.5mg,295.0mg,297.5mg,300.0mg,302.5mg,305.0mg,307.5mg,310.0mg,312.5mg, 315.0mg,317.5mg,320.0mg,322.5mg,325.0mg,327.5mg,330.0mg,332.5mg,335.0mg, 337.5mg,340.0mg,342.5mg,345.0mg,347.5mg,350.0mg,352.5mg,355.0mg,357.5mg, 360.0mg,362.5mg,365.0mg,367.5mg,370.0mg,372.5mg,375.0mg,377.5mg,380.0mg, 382.5mg,385.0mg,387.5mg,390.0mg,392.5mg,395.0mg,397.5mg,400.0mg,402.5mg, 405.0mg,407.5mg,410.0mg,412.5mg,415.0mg,417.5mg,420.0mg,422.5mg,425.0mg, 427.5mg,430.0mg,432.5mg,435.0mg,437.5mg,440.0mg,442.5mg,445.0mg,447.5mg, 450.0mg,452.5mg,455.0mg,457.5mg,460.0mg,462.5mg,465.0mg,467.5mg,470.0mg, 472.5mg,475.0mg,477.5mg,480.0mg,482.5mg,485.0mg,487.5mg,490.0mg,492.5mg, 495.0mg,497.5mg,500.0mg,502.5mg,505.0mg,507.5mg,510.0mg,512.5mg,515.0mg, 517.5mg.520.0mg.522.5mg.525.0mg.527.5mg.530.0mg.532.5mg.535.0mg.537.5mg. 540.0mg,542.5mg,545.0mg,547.5mg,550.0mg,552.5mg,555.0mg,557.5mg,560.0mg, 562.5mg,565.0mg,567.5mg,570.0mg,572.5mg,575.0mg,577.5mg,580.0mg,582.5mg, 585.0mg,587.5mg,590.0mg,592.5mg,595.0mg,597.5mg,600.0mg,602.5mg,605.0mg, 607.5mg,610.0mg,612.5mg,615.0mg,617.5mg,620.0mg,622.5mg,625.0mg,627.5mg, 630.0mg,632.5mg,635.0mg,637.5mg,640.0mg,642.5mg,645.0mg,647.5mg,650.0mg, 652.5mg,655.0mg,657.5mg,660.0mg,662.5mg,665.0mg,667.5mg,670.0mg,672.5mg, 675.0mg,677.5mg,680.0mg,682.5mg,685.0mg,687.5mg,690.0mg,692.5mg,695.0mg, 697.5mg,700.0mg,702.5mg,705.0mg,707.5mg,710.0mg,712.5mg,715.0mg,717.5mg, 720.0mg, 722.5mg, 725.0mg, 727.5mg, 730.0mg, 732.5mg, 735.0mg, 737.5mg, 740.0mg, 742.5mg,745.0mg,747.5mg,750.0mg,752.5mg,755.0mg,757.5mg,760.0mg,762.5mg, 765.0mg,767.5mg,770.0mg,772.5mg,775.0mg,777.5mg,780.0mg,782.5mg,785.0mg, 787.5mg,790.0mg,792.5mg,795.0mg,797.5mg or 800.0mg, administered once or twice a day.

In some embodiments, the administered dose of the compound shown in Formula (I) or a pharmaceutically acceptable salt thereof is selected from 50mg, 75mg, 100mg, 125mg, 150mg, 175mg, 200mg, 225mg, 250mg, 275mg, 300mg, 350mg, 375mg, 400mg, 425mg, 450mg, 475mg, 500mg, 525mg, 550mg, 575mg, or 600mg, administered once or twice a day.

In some embodiments, the administered dose of the compound shown in Formula (I) or a pharmaceutically acceptable salt thereof is selected from 50mg, 100mg, 200mg, 300mg, 350mg, or 400mg, administered once or twice a day.

In some embodiments, the administered dose of the compound shown in Formula (I) or a pharmaceutically acceptable salt thereof is selected from 200mg, 300mg, or 350mg, administered once or twice a day.

In some embodiments, the administered dose of the compound shown in Formula (I) or a pharmaceutically acceptable salt thereof is 200mg, administered once or twice a day.

In some embodiments, the administered dose of the compound shown in Formula (I) or a pharmaceutically acceptable salt thereof is 300mg, administered once or twice a day.

In some embodiments, the administered dose of the compound shown in Formula (I) or a pharmaceutically acceptable salt thereof is 350mg, administered once or twice a day.

In some embodiments, the administered dose of the compound shown in Formula (I) or a pharmaceutically acceptable salt thereof is selected from 200mg, 300mg, or 350mg, administered twice a day.

In some embodiments, the administered dose of the compound shown in Formula (I) or a pharmaceutically acceptable salt thereof is 200mg, administered twice a day.

In some embodiments, the administered dose of the compound shown in Formula (I) or a pharmaceutically acceptable salt thereof is 300mg, administered twice a day.

In some embodiments, the administered dose of the compound shown in Formula (I) or a pharmaceutically acceptable salt thereof is 350mg, administered twice a day.

Another aspect of the present disclosure provides a method of treating T-cell lymphoma, in which patients are given a therapeutically effective amount of a compound shown in Formula (I) or a pharmaceutically acceptable salt thereof.

Another aspect of the present disclosure provides a method of treating T-cell lymphoma, in which 1mg to 800mg of the compound shown in Formula (I) or a pharmaceutically acceptable salt thereof is administered to patients once or twice a day.

In some embodiments, the present disclosure provides a method of treating T-cell lymphoma, in which there is a 28-day dosing cycle.

Another aspect of the present disclosure provides a compound shown in Formula (I) for use in treating T-cell lymphoma, or a pharmaceutically acceptable salt thereof.

The term "patient" as used in the present disclosure means a human.

The acetylase inhibitors described in the present disclosure include but are not limited to chidamide, belimistat, or romidesin. The folic acid metabolism inhibitors include but are not limited to pralatrexate. The anti-CD30 antibodies include but are not limited to brentuximab vedotin.

Another aspect of the present disclosure provides a pharmaceutical composition comprising a compound shown in Formula (I) or a pharmaceutically acceptable salt thereof and one or more pharmaceutically acceptable carriers. The pharmaceutical composition may be specially formulated in solid or liquid form for oral administration or for local administration.

The present disclosure further provides the use of a composition comprising a compound shown in Formula (I) or a pharmaceutically acceptable salt thereof with one or more pharmaceutically acceptable carriers in the preparation of a medicament for the treatment of T-cell lymphoma.

"Pharmaceutically acceptable carrier" as described in this disclosure means any type of nontoxic inert solid, semi-solid or liquid filler, diluent, encapsulating material, or formulation excipient. Some examples of substances that may be pharmaceutically acceptable carriers are sugars, such as lactose or cellulose and derivatives thereof, such as sodium carboxymethyl cellulose, ethyl cellulose, and cellulose acetate.

In an optional embodiment, patients in the present disclosure have been treated with first-line chemotherapy and at least one new drug (chidamide, pralatrexate, brentuximab vedotin), wherein:
- CD30 systemic ALCL: Treated with brentuximab vedotin
- NKTCL: Treated with asparaginase/peraspartase regimen
- Other subtypes: Treated with CHOP or CHOP-like regimen (including but not limited to CHOEP, BV+CHP).

When relapsed/refractory is mentioned in the present disclosure, relapse refers to occurrence of progressive disease after the last-line treatment response, and refractory refers to failure of response of the last treatment.

ORR: Defined as the proportion of subjects with best efficacy of CR or PR from the start of the first dose to the occurrence of PD or the start of subsequent new anti-tumor treatment, whichever occurs first.

PFS: Defined as the date from the first dose to the first documented PD or death due to any cause, whichever occurs first. If the subject still does not experience PD or death as of the data cutoff date, the cutoff date is the date of the subject's last efficacy assessment. If the subject does not undergo tumor evaluation, the cut-off date is the date of first drug administration, and the PFS lasts for 1 day.

DoR: Defined as the time from the first assessment of CR or PR to the first assessment of PD or death due to any cause, whichever occurs first. If the subject still did not experience PD or death as of the data cut-off date, the cut-off date is the date of the subject's last efficacy assessment. Only for subjects who achieved CR or PR.

Progressive disease is defined as worsening of a subject's condition due to the indication under study. Including radiographic progression, progression of test results, and progression of clinical symptoms and signs. The appearance of new lesions or progression of existing lesions is considered to be progressive disease. Events that are life-threatening, require hospitalization or prolongation of hospitalization, or lead to permanent or severe disability/insufficiency/impact on work ability, congenital abnormalities, or birth defects due to the symptoms and signs of progressive disease should not be reported as SAEs. Deaths due to symptoms and signs of progressive disease should be reported as SAEs.

The "effective amount" or "effective therapeutic amount" in the present disclosure includes an amount sufficient to improve or prevent a symptom or disorder of a medical condition. An effective amount also means an amount sufficient to allow or facilitate diagnosis. The effective amount for a particular patient or veterinary subject can vary based on the following factors: These include the condition to be treated, the patient's general health, the route and dose of administration, and the severity of side effects. An effective amount can be the maximum dose or dosing regimen to avoid significant side effects or toxic effects.

### Specific Embodiment(s)

The following embodiments are used to further describe the present disclosure, but these embodiments are not intended to limit the scope of the present disclosure.

### Embodiment 1. Efficacy study of compound shown in Formula (I) in peripheral T-cell lymphoma

1.1 Drugs: Compound (WO2017084494A) shown in Formula (1), strength: 50mg/tablet, 200mg/tablet.
1.2 Methods of administration: 350mg, twice a day, 28 days as a cycle.
1.3 Inclusion criteria: Relapsed or refractory T lymphocytic lymphoma.

A total of 24 patients with relapsed or refractory PTCL were enrolled in the 350mg group, of whom 16 received chidamide, and all patients were analyzed for efficacy data based on indistinguishable prior treatment and prior chidamide treatment. See Table 1 for details.

**Table 1 Objective response rate by group**

| Dose group/previous treatment | | FAS | | | ES | | |
|---|---|---|---|---|---|---|---|
| 350mg group | Pathological subtype | ALL | PTCL-NOS | AITL | ALL | PTCL-NOS | AITL |
| | | (n=24) | (n=9) | (n=15) | (n=18) | (n=7) | (n=ll) |
| | CR (n) | 2 | 0 | 2 | 2 | 0 | 2 |
| | PR (n) | 10 | 4 | 6 | 10 | 4 | 6 |
| | ORR | 50% | 44.4% | 53.3% | 66.7% | 57.1% | 72.7% |
| 350mg group having used chidamide | Pathological subtype | ALL | PTCL-NOS | AITL | ALL | PTCL-NOS | AITL |
| | | (n=16) | (n=6) | (n=10) | (n=14) | (n=6) | (n=8) |
| | CR (n) | 1 | 0 | 1 | 1 | 0 | 1 |
| | PR (n) | 8 | 4 | 4 | 8 | 4 | 4 |
| | ORR | 56.3% | 66.7% | 50.0% | 64.3% | 66.7% | 62.5% |

24 patients with PTCL were enrolled, and the ORR was 50%, of whom, nine patients had PTCL-NOS, and 15 patients had AITL. The ORRs in the two groups were 44.4% and 53.3%, respectively. In the ES set, 18 patients had an ORR of 66.7%, of whom, seven patients had PTCL-NOS and 11 patients had AITL. The ORRs in the two groups was 57.1% and 72.7%, respectively.

Among the 24 patients with PTCL enrolled, 16 patients received chidamide, and the ORR was 56.3%, of whom, six patients had PTCL-NOS, and 10 patients had AITL. The ORRs in the two groups were 66.7% and 50.0%, respectively. In the ES set, 14 patients had an ORR of 64.3%, of whom, six patients had PTCL-NOS and eight patients had AITL. The ORRs in the two groups were 66.7% and 62.5%, respectively.

## Claims

1. A use of a compound shown in Formula (I) or a pharmaceutically acceptable salt thereof in the preparation of drugs for the treatment of T-cell lymphoma:

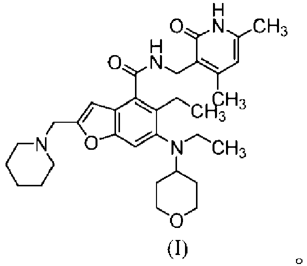

2. The use according to Claim 1, wherein the T-cell lymphoma is peripheral T-cell lymphoma (mature T and NK cell lymphoma), preferably angioimmunoblastic T-cell lymphoma, anaplastic large cell lymphoma, peripheral T lymphoma-non-specific or NK/T-cell lymphoma, most preferably angioimmunoblastic T-cell lymphoma or peripheral T lymphoma-non-specific.

3. The use according to Claim 2, wherein the peripheral T-cell lymphoma is relapsed/refractory peripheral T-cell lymphoma.

4. The use according to Claim 2, wherein the peripheral T-cell lymphoma is a peripheral T-cell lymphoma which has undergone first-line chemotherapy and/or at least one treatment selected from histone deacetylase inhibitors, folic acid metabolism inhibitors, or anti-CD30 monoclonal antibodies. Preferably, the peripheral T-cell lymphoma is a peripheral T-cell lymphoma which has undergone first-line chemotherapy, or first-line chemotherapy and at least one treatment selected from histone deacetylase inhibitors, folic acid metabolism inhibitors, or anti-CD30 monoclonal antibodies.

5. The use according to any one of Claims 1-4, wherein the administered dose of the compound shown in Formula (I) or a pharmaceutically acceptable salt thereof is selected from 1mg to 800mg, administered once or twice a day.

6. The use according to Claim 5, wherein the administered dose of the compound shown in Formula (I) or a pharmaceutically acceptable salt thereof is selected from 50mg, 75mg, 100mg, 125mg, 150mg, 175mg, 200mg, 225mg, 250mg, 275mg, 300mg, 350mg, 375mg, 400mg, 425mg, 450mg, 475mg, 500mg, 525mg, 550mg, 575mg, or 600mg, administered once or twice a day. Preferably, the administered dose of the compound shown in Formula (I) or a pharmaceutically acceptable salt thereof is selected from 50mg, 100mg, 200mg, 300mg, 350mg, or 400mg, administered once or twice a day. Most preferably, the administered dose of the compound shown in Formula (I) or a pharmaceutically acceptable salt thereof is selected from 200mg, 300mg, or 350mg, administered once or twice a day.
